# EUROPEAN PATENT SPECIFICATION

(11) **EP 4 201 334 B1**
(45) Date of publication and mention of the grant of the patent: **16.07.2025**
(21) Application number: 22216583.9
(22) Date of filing: 23.12.2022
(51) Int. Cl.: A61B 6/00, G01N 23/046, G01N 23/10, G01T 7/00

(54) **CALIBRATION ASSEMBLY, CALIBRATION PHANTOM AND CALIBRATION METHOD**
KALIBRIERUNGSANORDNUNG, KALIBRIERUNGSPHANTOM UND KALIBRIERUNGSVERFAHREN
ENSEMBLE D'ÉTALONNAGE, FANTÔME D'ÉTALONNAGE ET PROCÉDÉ D'ÉTALONNAGE

(30) Priority: 27.12.2021 CN 202111618227
(43) Date of publication of application: 28.06.2023
(73) Proprietor: Nuctech Company Limited, TongFang Building Shuangqinglu Road Haidian District Beijing 100084 (CN); Tsinghua University, Haidian District, Beijing 100084 (CN)
(72) Inventor: ZHANG, Li, BEIJING, 100084 (CN); CHEN, Zhiqiang, BEIJING, 100084 (CN); SUN, Yunda, BEIJING, 100084 (CN); LI, Xinbin, BEIJING, 100084 (CN); ZHAO, Zhenhua, BEIJING, 100084 (CN); ZHAO, Tiao, BEIJING, 100084 (CN); JIN, Xin, BEIJING, 100084 (CN)
(74) Representative: Cabinet Beau de Loménie

(56) References cited:
- GB-A- 621 107
- US-A- 4 613 754
- US-A1- 2003 095 693
- US-A1- 2006 239 414
- US-A1- 2021 223 188

## Description

### TECHNICAL FIELD

At least one embodiment of the present disclosure relates to a calibration phantom for calibrating a CT imaging system, and in particular to a calibration assembly, a calibration phantom, and a calibration method for calibrating a CT imaging system before the CT imaging system performs a scanning operation.

### BACKGROUND

The CT (Computed Tomography) imaging technology is currently widely used in a plurality of fields such as medical treatment and security inspection, which may acquire a three-dimensional image information of an internal structure of a detected object or person without damage. In a process of CT imaging, a geometric error of a CT imaging system may cause a structure distortion of a reconstructed three-dimensional image, resulting in problems such as inaccurate CT values or ring artifacts. The CT imaging system generally includes an X-ray machine, an object to be imaged and a detector, and the reconstruction image is generally obtained by adjusting a geometric parameter of the X-ray machine or the detector.

Before scanning and imaging, it is generally necessary to calibrate the CT imaging system by using a calibration phantom. By continuously adjusting a geometric parameter value in an imaging method, an optimal geometric parameter value closest to a real scanning system structure may be obtained. The existing geometric calibration methods mainly aim at a spiral scanning cone beam CT apparatus, and determine a method and an effect of member adjustment by manually or electrically adjusting some members on the CT apparatus relying on human subjective judgments or objective indicators. The above-mentioned adjustment devices and adjustment methods are generally time-consuming and laborious, and are greatly affected by subjective factors. Geometric parameters of the existing CT imaging systems are numerous and complex, and the optimal image reconstruction effect may not be obtained through geometric calibration devices and calibration methods in the related art.

The following is related art to the present disclosure.

US Patent Application Publication No. US2021223188A1 discloses a calibration assembly and a calibration method of calibrating geometric parameters of a CT apparatus. The calibration assembly includes at least one calibration unit each including a plurality of calibration wires, and the plurality of calibration wires are arranged regularly in a same plane. The calibration assembly is easy to be processed and can be used to calibrate geometric parameters of a CT apparatus, and the calibration operations are simple and easy to be implemented.

US Patent Application Publication No. US2003095693A1 discloses methods and devices for improving the machine-to-machine and temporal (e.g., inter and intra-machine) and database consistency of coronary calcium scoring by applying a filtering algorithm that sharpens and/or smoothes the image so as to return a filtered image having a spatial resolution of a certain reference value.

US Patent Application Publication No. US4613754A discloses an apparatus for providing data for the calibration of the atomic number and density of a test material being scanned in the radiation field of a computerized axial tomographic scanner (CAT). The apparatus comprises a housing which is adapted to be positioned in the X-ray field of the CAT in a predetermined position. The housing has an aperture that is adapted to accommodate the test material, and a plurality of calibration materials which have known atomic numbers and densities are positioned in the housing. In addition, the present invention provides a calibration apparatus that has a surface that is adapted to accommodate the test material.

US Patent Application Publication No. US2006239414A1 discloses a phantom for the quality control of a radiotherapy treatment virtual simulation system which comprises a medical imaging device, characterized in that it comprises: a support casing, a core which is arranged in the support casing and which is constituted by a plurality of elements of different shapes, dimensions and densities, the densities simulating the densities of various organs and media of the human body, two of these elements being constituted by two truncated pyramids of different densities which are fitted on inside the other, at least one of them not being completely symmetrical relative to the longitudinal axis thereof, balls of a non-radiotransparent material arranged in the core, at least two removable lateral faces which face each other and which comprise metal wires which define geometric figures.

British Patent Application Publication No. GB621107A discloses improvements in methods of estimating depth in stereoscopic X-ray photographs or, as they are termed in the art, stereoscopic radiographs. It is desired, however, that the scope of the invention shall also be regarded as including the application of the principle to all types of stereoscopic still or moving pictures which do not necessitate the use of individual viewing devices by the observers, whether such pictures be viewed directly or after projection onto a screen.

### SUMMARY

The subject-matter for which protection is sought is defined by the appended claims.

In view of at least one technical problem of the above or other aspects of the prior art, the embodiments of the present disclosure provide a calibration component, a calibration phantom, and a calibration method, which may improve a calibration accuracy of a CT imaging system.

According to a first aspect of the present disclosure, a calibration assembly is provided. The calibration assembly includes a base and a plurality of calibration wires dispersedly connected to the base. An absorption capacity of the calibration wire for X-rays is greater than an absorption capacity of the base for X-rays.

According to the embodiments of the present disclosure, the calibration assembly further includes two mounting portions arranged at two opposite ends of the base.

According to the embodiments of the present disclosure, shapes of sections of the calibration wires are set as one or more of approximately circular, elliptical, rectangular, square and polygon.

According to the embodiments of the present disclosure, a diameter of a smallest circle enclosing a section of the calibration wire falls between 0.5mm and 5 mm. For example, the circle may be the circumscribed circle of the section if the section is a polygon, or may coincide with the entire outer edge of the section if the section is a circle.

According to the embodiments of the present disclosure, the base has a flat plate shape, and the calibration wires are arranged on the base in a length direction of the base.

According to the embodiments of the present disclosure, a spacing among adjacent calibration wires is equal and/or unequal.

According to the embodiments of the present disclosure, at least one end of the calibration wire protrudes from a side of the base.

According to the embodiments of the present disclosure, the base includes two sub-bases arranged in parallel, and the calibration wire is connected between the two sub-bases.

According to a second aspect of the present disclosure, a calibration phantom is provided. The calibration phantom includes a supporting frame and at least one calibration assembly as described above. The at least one calibration assembly is connected to an inside of the supporting frame, and an absorption capacity of the calibration wire for X-rays is greater than an absorption capacity of the supporting frame for X-rays.

According to the embodiments of the present disclosure, each calibration assembly is connected to the inside of the supporting frame through mounting portions arranged at two ends of the calibration assembly.

According to the embodiments of the present disclosure, the supporting frame is set as a substantially hexahedral box; and wherein at least one calibration assembly is arranged in a length direction of the supporting frame.

According to the embodiments of the present disclosure, projections of calibration wires of each calibration assembly in a height direction and/or a length direction of the supporting frame do not coincide with each other.

According to the embodiments of the present disclosure, projections of at least two calibration assemblies arranged in the length direction of the supporting frame in the length direction do not coincide with each other.

According to the embodiments of the present disclosure, projections of the at least two calibration assemblies arranged in the length direction of the supporting frame in the height direction do not coincide with each other.

According to the embodiments of the present disclosure, projections of at least two calibration assemblies in a transverse direction of the supporting frame intersect with each other, and projections of the at least two calibration assemblies in the length direction of the supporting frame do not coincide with each other.

According to a third aspect of the present disclosure, a calibration method is provided, including: placing the calibration phantom as described above in a scanning channel of a scanning system; performing X-ray scanning on the calibration phantom to obtain an imaging result of the calibration phantom; and evaluating the imaging result according to a preset evaluation method to obtain a first evaluation result. The method further includes: completing a calibration of the scanning system in response to the first evaluation result meeting a preset condition; or, adjusting, in response to the first evaluation result not meeting the preset condition, a geometric parameter value of a preset system according to the first evaluation result, and repeating the step of evaluating the imaging result according to the preset evaluation method.

According to the embodiments of the present disclosure, repeating the step of evaluating the imaging result includes: evaluating the imaging result according to the preset evaluation method to obtain a second evaluation result. The method further includes: completing the calibration of the scanning system in response to the second evaluation result meeting the preset condition; or, adjusting, in response to the second evaluation result not meeting the preset condition, the geometric parameter value of the preset system according to a reference evaluation result, and repeating the step of evaluating the imaging result according to the preset evaluation method, wherein the reference evaluation result is characterized as a comparison value between the first evaluation result and the second evaluation result adjacent to the first evaluation result.

According to the embodiments of the present disclosure, after the step of performing X-ray scanning on the calibration phantom to obtain an imaging result of the calibration phantom, the method further includes: simulating a numerical model of the calibration phantom according to the geometric parameter value of the preset system to obtain a simulation result; and comparing the simulation result with the imaging result to obtain a comparison data. The method further includes: completing the calibration of the scanning system in response to the comparison data meeting the preset condition; or, adjusting, in response to the comparison data not meeting the preset condition, the geometric parameter value of the preset system according to the comparison data, and repeating the step of simulating a numerical model of the calibration phantom according to the geometric parameter value of the preset system.

According to the embodiments of the present disclosure, a length direction of the calibration phantom is perpendicular to a scanning direction of the calibration phantom.

According to the embodiments of the present disclosure, the step of performing X-ray scanning on the calibration phantom includes: scanning the calibration phantom once in response to a size of the calibration phantom being close to a size of the scanning channel of the scanning system; or, changing a position of the calibration phantom in the scanning channel and scanning the calibration phantom for a plurality of times in response to the size of the calibration phantom being smaller than the size of the scanning channel of the scanning system.

According to the embodiments of the present disclosure, the parameter includes a relative position and a posture between a light source and a detector in the scanning system.

According to the embodiments of the present disclosure, the step of simulating a numerical model of the calibration phantom according to the geometric parameter value of the preset system is implemented by using one or more of a pure parameter fitting method, a digital radiography (hereinafter "DR") modeling projection simulation and a CT reconstructed projection simulation.

According to the embodiments of the present disclosure, the simulation result includes one or more of a projection domain simulation result, a projection domain parametric simulation result, an image domain simulation result and an image domain parametric simulation result.

According to the embodiments of the present disclosure, the imaging result includes one or more of a scanning data, a DR image, a slice image and a three-dimensional reconstructed image.

According to the calibration wire, the calibration phantom and the calibration method of the above-mentioned embodiments of the present disclosure, by dispersedly connecting the calibration wires of the calibration component to the base, and taking advantage of the characteristics that the absorption capacity of the calibration wire for X-rays is greater than the absorption capacity of the base for X-rays, the above-mentioned calibration wires are applied to the calibration phantom, and the calibration phantom is scanned in the scanning system. By continuously adjusting the geometric parameter values in the imaging method, the optimal geometric parameter values that are closest to the real scanning system structure may be obtained, thereby improving the imaging effect of the scanning system.

### BRIEF DESCRIPTION OF THE DRAWINGS

The above contents and other objectives, features and advantages of the present disclosure will be more apparent through the following descriptions of embodiments of the present disclosure with reference to the accompanying drawings.
FIG. 1a schematically shows a schematic stereoscopic diagram of a calibration assembly according to an exemplary embodiment of the present disclosure.
FIG. 1b schematically shows a side view of the calibration assembly shown in FIG. 1a.
FIG. 2 schematically shows a schematic stereoscopic diagram of a calibration assembly according to another exemplary embodiment of the present disclosure.
FIG. 3 schematically shows a schematic stereoscopic diagram of a calibration assembly according to yet another exemplary embodiment of the present disclosure.
FIG. 4a schematically shows a schematic stereoscopic diagram of a calibration assembly according to still another exemplary embodiment of the present disclosure.
FIG. 4b schematically shows a side view of a calibration assembly according to still another exemplary embodiment of the present disclosure.
FIG. 4c schematically shows another calibration wire arrangement of the calibration assembly shown in FIG. 4a.
FIG. 5 schematically shows a side view of a calibration assembly according to still another exemplary embodiment of the present disclosure.
FIG. 6 schematically shows a schematic stereoscopic diagram of a calibration phantom according to an embodiment of the present disclosure.
FIG. 7 shows a simplified schematic diagram of a CT scanning apparatus according to an exemplary embodiment of the present disclosure.
FIG. 8 shows a schematic principle diagram of a CT imaging system according to an exemplary embodiment of the present disclosure.
FIG. 9 to FIG. 11 schematically show flow charts of calibration methods according to the embodiments of the present disclosure.

### [Reference Numerals]

100 - calibration assembly;
101 - base;
102 - calibration wire;
1021 - calibration wire group;
103 - mounting portion;
200 - supporting frame;
1 - X-ray source;
2 - detector array;
3 - slip ring;
4 - conveyor;
5 - detected target;
6 - scanning channel;
10 - CT imaging system.

### DETAILED DESCRIPTION OF EMBODIMENTS

Embodiments of the present disclosure will be described below with reference to the accompanying drawings. However, it should be understood that these descriptions are merely exemplary and are not intended to limit the scope of the present disclosure. In the following detailed descriptions, for purposes of explanation, numerous specific details are set forth in order to provide a comprehensive understanding of the embodiments of the present disclosure. However, it is obvious that one or more embodiments may be implemented without these specific details. In addition, in the following descriptions, descriptions of well-known structures and technologies are omitted to avoid unnecessarily obscuring the concept of the present disclosure.

Terms used herein are for the purpose of describing specific embodiments only and are not intended to limit the present disclosure. Terms such as "comprising", "including" and the like used herein specify a presence of a feature, a step, an operation and/or a component, but do not preclude a presence or addition of one or more other features, steps, operations or components.

All terms (including technical and scientific terms) used herein have the same meaning as commonly understood by those skilled in the art, unless otherwise defined. It should be noted that the terms used herein should be construed as having meanings consistent with the context of the present description and should not be construed in an idealized or overly rigid manner.

Where expressions like "at least one of A, B, and C, etc." are used, they should generally be interpreted in accordance with the meaning of the expression as commonly understood by those skilled in the art (e.g., "a system having at least one of A, B and C" should include, but not be limited to, a system having A alone, having B alone, having C alone, having A and B, having A and C, having B and C, and/or having A, B, C, etc.).

According to a general inventive concept of the present disclosure, there is provided a calibration assembly, including: a base and a plurality of calibration wires dispersedly connected to the base, wherein an absorption capacity of the calibration wires for X-rays is greater than an absorption capacity of the base for X-rays.

According to another general inventive concept of the present disclosure, there is provided a calibration phantom, including: a supporting frame and at least one calibration assembly described above connected to an inside of the supporting frame, wherein an absorption capacity of the calibration wires for X-rays is greater than an absorption capacity of the supporting frame for X-rays.

According to yet another inventive concept of the present disclosure, there is provided a calibration method, including: placing the calibration phantom described above in a scanning channel of a scanning system; performing X-ray scanning on the calibration phantom to obtain an imaging result of the calibration phantom; and evaluating the imaging result according to a preset evaluation method to obtain a first evaluation result. The method further includes: completing a calibration of the scanning system in response to the first evaluation result meeting a preset condition; or, adjusting, in response to the first evaluation result not meeting the preset condition, a geometric parameter value of a preset system according to the first evaluation result, and repeating the step of evaluating the imaging result according to the preset evaluation method.

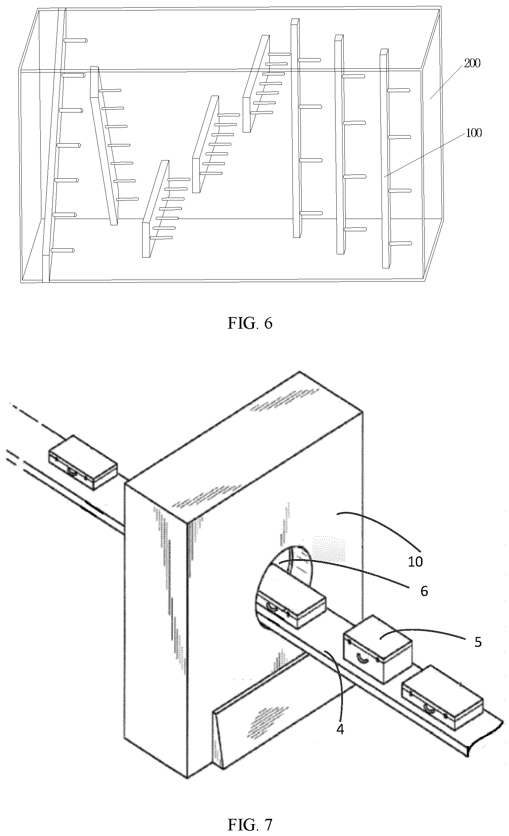

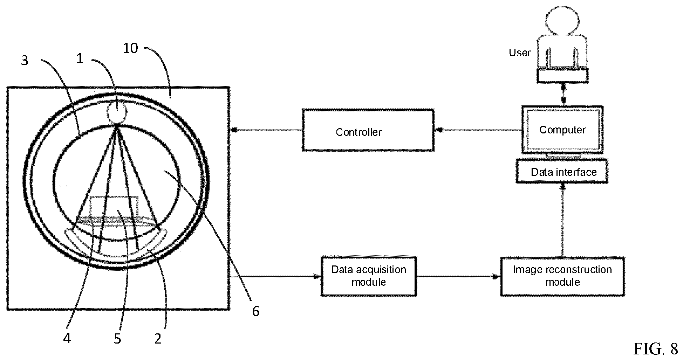

FIG. 7 shows a simplified schematic diagram of a CT scanning apparatus according to an exemplary embodiment of the present disclosure. FIG. 8 shows a schematic principle diagram of a CT imaging system according to an exemplary embodiment of the present disclosure.

A CT imaging system 10 mainly includes a supporting frame, a slip ring (rotating gantry) 3 rotatably mounted on the supporting frame, an X-ray source 1 and a detector array 2 mounted on the slip ring 3. During a process of inspecting an inspected object 5, a controller receives an operation command input by a user through a computer at a workstation, and controls a driving mechanism to act according to the operation command. The slip ring 3 drives the X-ray source 1 and the detector array 2 to rotate under a driving of the driving mechanism. At the same time, the X-ray source 1 may generate an X-ray beam under a control of the controller. The X-ray beam penetrates the inspected target 5 on a conveyor 4 moving in an inspection channel 6 and irradiates onto the detector array 2. The detector array 2 converts the received X-ray beam into an electrical signal and transmits the electrical signal to a data acquisition module. An image reconstruction module receives data of the data acquisition module, reconstructs the received data and generates image data. The generated image data is transmitted to the computer so as to identify and inspect the inspected object. Generally, before scanning and imaging, it is usually necessary to calibrate the CT imaging system by using a calibration phantom. By continuously adjusting a geometric parameter value in an imaging method, an optimal geometric parameter value closest to a real scanning system structure may be obtained, thereby improving an imaging effect of the CT imaging system.

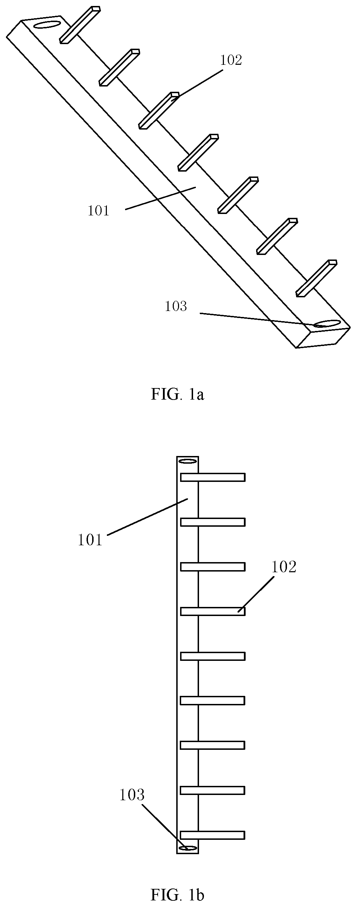

FIG. 6 schematically shows a schematic stereoscopic diagram of a calibration phantom according to an embodiment of the present disclosure. FIG. 1a schematically shows a schematic stereoscopic diagram of a calibration assembly according to an exemplary embodiment of the present disclosure. FIG. 1b schematically shows a side view of the calibration assembly shown in FIG. 1a.

Referring to FIG. 6, according to an exemplary embodiment of the present disclosure, there is provided a calibration phantom, including a calibration assembly 100 and a supporting frame 200.

In an embodiment, as shown in FIG. 1a and FIG. 1b, the calibration assembly 100 includes a base 101 and a plurality of calibration wires 102. The calibration wires 102 are dispersedly connected to the base 101, and an absorption capacity of the calibration wire 102 for X-rays is greater than an absorption capacity of the base 101 for X-rays.

In an embodiment, the calibration assembly 100 further includes two mounting portions 103 arranged at two opposite ends of the base 101.

The calibration wire 102 may be made of materials having a strong X-ray absorption capacity, such as one of iron, copper, lead or cadmium or a combination thereof. For example, the calibration wire is made of steel wire. The base 101 may be made of materials having a weak X-ray absorption capacity, such as organic glass, acrylic, resin or the like. By selecting the above-mentioned materials for the calibration wire 102 and the base 101, the calibration wire 102 may be very concentrated in a reconstructed image without divergence, so that the reconstructed image of the calibration assembly in the scanning system may be clearly distinguished from a background region, and thereby it is easy to determine whether the reconstructed image of the calibration wire 102 is good or not.

As shown in FIG. 1a and FIG. 1b, a shape of the calibration wire 102 in the embodiment of the present disclosure is a long strip, but the shape of the calibration wire 102 is not limited to this, and may be approximately a cylinder, a prism, a triangle, or other irregular columnar bodies, which will not be illustrated here. A shape of a section of the calibration wire may be approximately to a circle, an ellipse, a rectangle, a square, a polygon or other irregular shapes.

Generally, the shape of the calibration wire 102 is approximate to a cylinder. In order to ensure that the reconstructed image of the calibration assembly in the scanning system corresponds to a small number of detector pixels, the diameter of the calibration wire 102 may be made as small as possible. For example, the diameter of the calibration wire 102 may fall within the range between 0.5mm and 5 mm (0.5mm and 5 mm included), preferably between 1mm and 2 mm.

As shown in FIG. 1a, the base 101 is formed as a flat plate shape, and the calibration wires 102 are arranged on the base 101 in a length direction of the base 101.

Specifically, in the length direction, a spacing between two adjacent calibration wires 102 is generally not specifically limited. For example, in order to facilitate processing and calibration, the calibration wires 102 may be spaced by equal spacing on the base 101 in the length direction.

As a specific embodiment of the present disclosure, at least one end of the above-mentioned calibration wires 102 protrudes from a side of the base 101, and protruded lengths thereof are approximately the same.

As shown in FIG. 1a and FIG. 1b, the calibration wires 102 protrude from the same side of the base 101.

Optionally, one end of a single calibration wire 102 may be fixedly connected to the base 101.

Optionally, the left end of the calibration wire 102 may be fixedly connected to the base 101, and the plurality of calibration wires 102 are arranged in the length direction of the base 101. It should be noted here that: in order to facilitate processing and calibration, adjacent calibration wires 102 may be spaced by equal spacing, or may be unequally spaced according to actual calibration requirements, which will not be specifically limited herein.

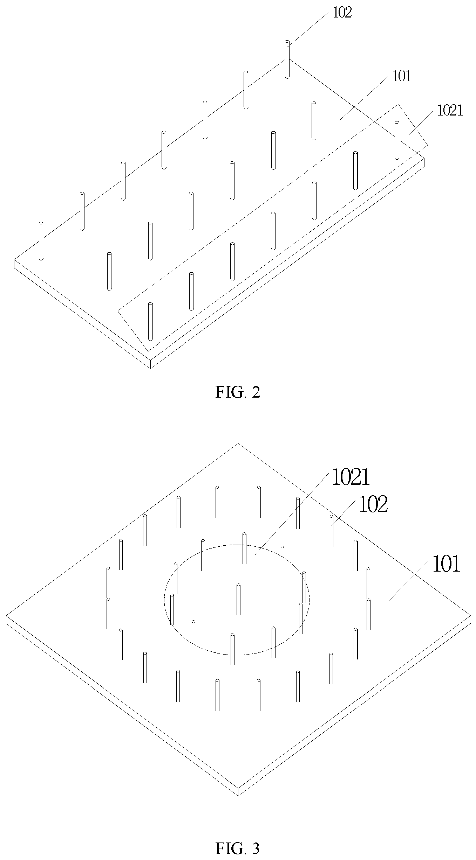

Optionally, as shown in FIG. 2, the plurality of calibration wires 102 are spaced by the equal spacing in the length direction of the base 101 as a calibration wire group 1021, and a plurality of calibration wire groups 1021 are arranged in a width direction of the base 101. It should be noted here that the spacing among the plurality of calibration wires 102 or among the plurality of calibration wire groups 1021 may be equal, or may be unequal according to actual calibration requirements, which will not be specifically limited herein.

Optionally, as shown in FIG. 3, the plurality of calibration wires 102 are arranged on the base 101 in a circumferential direction as a calibration wire group 1021, and a plurality of calibration wire groups 1021 are arranged in a radial direction as a plurality of rings. It should be noted here that the spacing among the plurality of calibration wires 102 or among the plurality of calibration wire groups 1021 may be equal, or may be unequal according to actual calibration requirements, which will not be specifically limited herein.

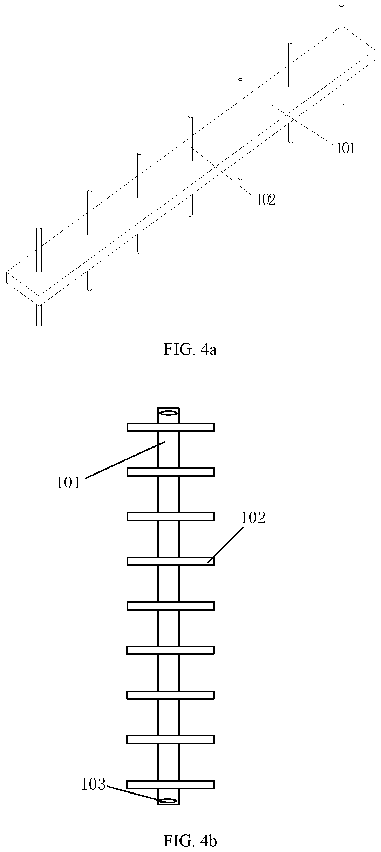

FIG. 4a schematically shows a schematic stereoscopic diagram of a calibration assembly according to still another exemplary embodiment of the present disclosure. FIG. 4b schematically shows a side view of a calibration assembly according to still another exemplary embodiment of the present disclosure. As shown in FIG. 4a and FIG. 4b, the calibration wires 102 protrude from two sides of the base 101.

Optionally, a middle portion of the calibration wire 102 may be fixedly connected to the base 101, and the plurality of calibration wires 102 are arranged in the length direction of the base 101. Spacing among adjacent calibration wires 102 may be equal or unequal, and may be set according to actual calibration requirements, which will not be specifically limited herein.

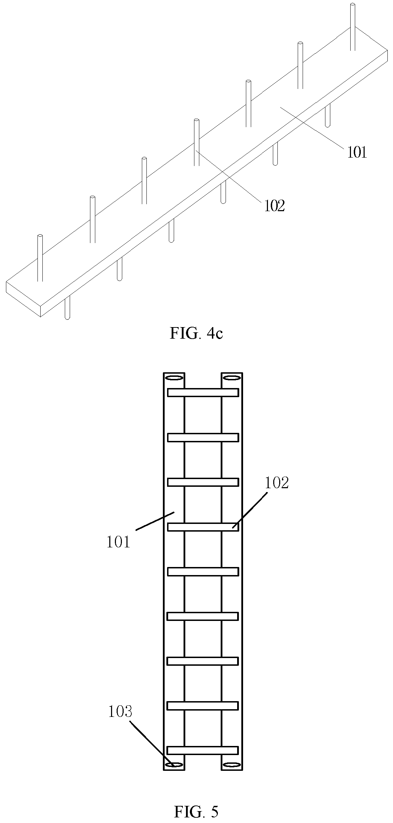

FIG. 4c schematically shows another calibration wire arrangement of the calibration assembly shown in FIG. 4a.

As shown in FIG. 4c, axes of the calibration wires 102 fixedly connected to the two sides of the base 101 are provided in a non-coaxial way. Specifically, one end of the calibration wires 102 may be fixedly connected to upper and lower edge surfaces of the base 101, respectively, and the plurality of calibration wires 102 are arranged in the length direction of the base 101.

It should be noted here that the axis of the calibration wire 102 fixedly connected to the upper edge surface of the base 101 and the axis of the calibration wire 102 fixedly connected to the lower edge surface of the base 101 may be coaxial, as shown in FIG. 4a and FIG. 4b, and may also not be coaxial, as shown in FIG. 4c, and may be set according to actual calibration requirements, which will not be specifically limited herein.

It should also be noted here that the spacing among adjacent calibration wires 102 may be equal or unequal, and may be set according to actual calibration requirements, which will not be specifically limited herein.

FIG. 5 schematically shows a side view of a calibration assembly according to still another exemplary embodiment of the present disclosure. As shown in FIG. 5, two ends of the calibration wire 102 may be respectively fixed on two sub-bases 101. Optionally, the plurality of calibration wires 102 are spaced by the equal spacing in the length direction of the base 101.

The connection manner of the calibration wires 102 on the base 101 includes but is not limited to the connection manner shown in FIG. 1a to FIG. 5. For example, the calibration wires arranged in rows may be arranged on the base in a same inclined manner.

As shown in FIG. 6, another embodiment of the present disclosure further discloses a calibration phantom, including: a supporting frame 200 and at least one calibration assembly 100 described above. The calibration assembly 100 is connected to an inside of the supporting frame 200 through the mounting portion 103. For example, the mounting portion 103 may be connected with the supporting frame 200 by a screw.

The absorption capacity of the calibration wire 102 for X-rays is greater than the absorption capacity of the supporting frame 200 for X-rays.

The number of calibration assemblies may be set according to calibration requirements, which will not be specifically limited, and may be, for example, 1, 2, 3, 4, 5, 6, 7, 8, or the like.

The supporting frame 200 may be set as a substantially hexahedral box.

In some embodiments where at least two calibration assemblies are provided, the calibration assemblies may be arranged in a length direction of the supporting frame 200. In some embodiments where only one calibration assembly is provided, a position where the calibration assembly is provided is not specifically limited, as long as a calibration task may be completed.

As shown in FIG. 6, each calibration assembly 100 is staggered in an internal space of the supporting frame 200, and projections of the calibration wires 102 of each calibration assembly 100 in a height direction and/or a length direction of the supporting frame 200 do not coincide with each other. As shown in FIG. 6, in the embodiment, eight groups of calibration assemblies 100 are connected to the inside of the supporting frame 200, and connection positions and postures of each calibration assembly 100 on the supporting frame 200 are different from each other. Thus, each calibration assembly 100 may be clearly imaged in the scanning system, and the calibration wires 102 in each calibration assembly 100 do not coincide with each other, which is convenient for the calibration assembly to be clearly imaged, and may obtain a true value of the calibration phantom imaging more accurately and conveniently.

In an exemplary embodiment, as shown in FIG. 6, projections of at least two calibration assemblies arranged in the length direction of the supporting frame 200 in the length direction do not coincide with each other. In another exemplary embodiment, projections of at least two calibration assemblies arranged in the length direction of the supporting frame 200 in the height direction do not coincide with each other.

According to the embodiments of the present disclosure, projections of at least two calibration assemblies in a transverse direction of the supporting frame 200 intersect with each other and projections of at least two calibration assemblies in the length direction of the supporting frame 200 do not coincide with each other.

The above-mentioned embodiments relate to the "non-coincidence" arrangement of projections of the calibration assemblies in different directions, which is mainly for ease of calibration. However, in some embodiments, it is possible for the calibration assemblies to coincide with each other. For example, the calibration wires are arranged in two dimensions, and some calibration wires may coincide at some certain angles, but the calibration wires may also be reconstructed. For another example, it is possible that projections on different angles may have a calibration wire projection coincidence, which may also be suitable for calibration.

It may be understood that the calibration wires may be arranged to be horizontal, vertical, left-inclined, and right-inclined with respect to the base 101. There may be one or more groups of calibration wires in the calibration phantom. Each group of calibration wires may be placed horizontally, vertically, leftward obliquely or rightward obliquely with respect to the length direction of the calibration phantom. Height positions of each group of calibration wires may also be varied.

In order to obtain a better calibration effect, the calibration phantom in the CT scanning system should be a simple and easily distinguishable object so as to facilitate evaluating the reconstruction effect in the reconstructed image of the CT scanning system. Thus, effective projection information may be obtained from projection data of the calibration phantom as a "true value" of the target in the calibration process, so that a calibrated result may be as close to the "true value" as possible. In addition, the calibration phantom should cover an entire scanning region or a designated calibration region of the CT scanning system as much as possible, so that the effect of the reconstructed image within the region after calibration may reach an overall optimization, and a significant image quality degradation of the reconstructed image in some certain regions may be avoided.

As shown in FIG. 7 and FIG. 8, a multi-view scanning imaging manner may be implemented for the calibration phantom, that is, a fabricated calibration phantom instead of the inspected object 5 is placed on the conveyor 4 (such as a conveyor belt) moving in the scanning channel 6 of the CT scanning system, so that the calibration phantom moves in the scanning channel 6 with the conveyor 4, and the X-ray source 1 may generate X-ray beams under the control of the controller to achieve the scanning projection imaging under multiple imaging viewing angles. If an overall size of the calibration phantom is small, the calibration phantom may be placed in different positions and different postures in the scanning channel 6, so that a distribution range of the calibration wires 102 covers a main imaging region or the entire scanning channel. From a perspective of parameter optimization, the more scanning projection data of the calibration wire 102, the larger the distribution range in the scanning channel 6, and the more uniform the distribution interval, the greater a tolerance of the calibration method to data error, and geometric parameters obtained after calibrating the corresponding projection data are also closer to true parameter values, and the effect of the reconstructed three-dimensional image is better.

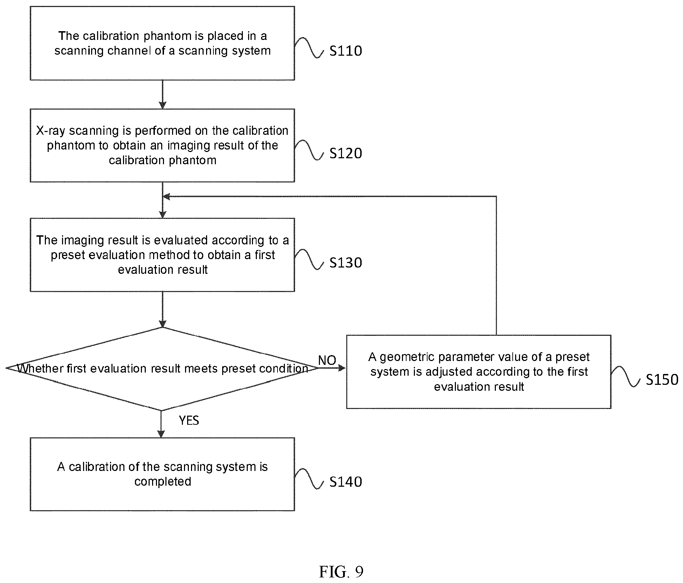

In order to describe the scanning process of the scanning system in more detail, the scanning process is described below with reference to FIGS. 9-11.

FIG. 9 schematically shows a flow chart of a calibration method according to the embodiments of the present disclosure.

As shown in FIG. 9, another embodiment of the present disclosure further discloses a calibration method, including the following steps S110 to S150.

Step S110: the calibration phantom described above is placed in a scanning channel of a scanning system.

Step S120: X-ray scanning is performed on the calibration phantom to obtain an imaging result of the calibration phantom, wherein the imaging result may be selected from scanning data, a DR image, a slice image and a three-dimensional reconstructed image.

Step S130: the imaging result is evaluated according to a preset evaluation method to obtain a first evaluation result.

Step S140: a calibration of the scanning system is completed in response to the first evaluation result meeting a preset condition.

Step S150: a geometric parameter value of a preset system is adjusted according to the first evaluation result in response to the first evaluation result not meeting the preset condition, and the method is returned to step S130.

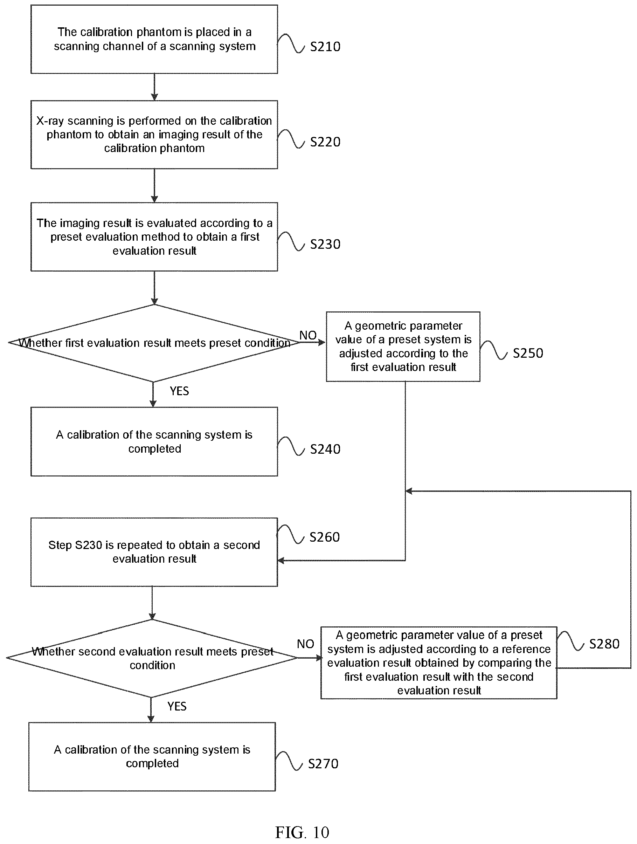

FIG. 10 schematically shows a flowchart of a calibration method according to the embodiments of the present disclosure.

As shown in FIG. 10, another embodiment of the present disclosure further discloses a calibration method, including the following steps S210 to S280.

Step S210: the calibration phantom described above is placed in a scanning channel of a scanning system.

Step S220: X-ray scanning is performed on the calibration phantom to obtain an imaging result of the calibration phantom, wherein the imaging result may be selected from scanning data, a DR image, a slice image and a three-dimensional reconstructed image.

Step S230: the imaging result is evaluated according to a preset evaluation method to obtain a first evaluation result.

Step S240: a calibration of the scanning system is completed in response to the first evaluation result meeting a preset condition.

Step S250: a geometric parameter value of a preset system is adjusted according to the first evaluation result in response to the first evaluation result not meeting the preset condition, and the method proceeds to step S260.

Step S260: step S230 is repeated to obtain a second evaluation result.

Step S270: the calibration of the scanning system is completed in response to the second evaluation result meeting the preset condition.

Step S280: the geometric parameter value of the preset system is adjusted according to a reference evaluation result in response to the second evaluation result not meeting the preset condition, and the method is returned to step S260.

The reference evaluation result is characterized as a comparison value between the first evaluation result and the second evaluation result adjacent to the first evaluation result. Thus, the reference evaluation result is generally a comparison value between evaluation results obtained from two adjacent cycles, such as a comparison value between the second evaluation result and the third evaluation result, a comparison value between the third evaluation result and the fourth evaluation result or the like.

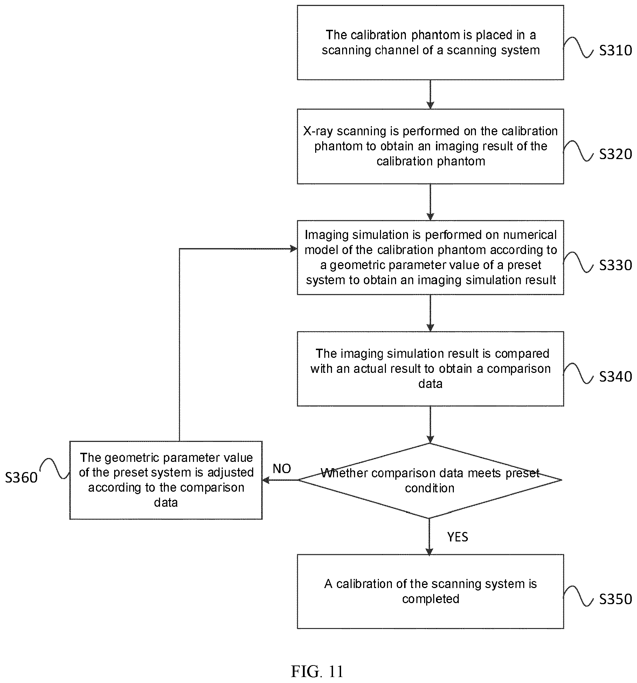

FIG. 11 schematically shows a flowchart of a calibration method according to the embodiments of the present disclosure.

As shown in FIG. 11, another embodiment of the present disclosure further discloses a calibration method, including the following steps S310 to S360.

Step S310: the calibration phantom described above is placed in a scanning channel of a scanning system.

Step S320: X-ray scanning is performed on the calibration phantom to obtain an imaging result of the calibration phantom, wherein the imaging result may be selected from scanning data, a DR image, a slice image or a three-dimensional reconstruction image.

Step S330: a numerical model of the calibration phantom is simulated according to a geometric parameter value of a preset system to obtain a simulation result, wherein the simulation result may be selected from a projection domain simulation result, a projection domain parametric simulation result, an image domain simulation result, and an image domain parametric simulation result.

Specifically, the geometric parameter value of the preset system may be a geometric parameter value of an existing system or a geometric parameter value of an adjusted system.

The simulation method may be selected from one or more of a pure parameter fitting method, a DR modeling projection simulation and a CT reconstructed projection simulation.

Step S340: the simulation result is compared with an actual result to obtain comparison data. It should be noted that the actual result may be a DR image, a slice image or a three-dimensional image.

Step S350: a calibration of the scanning system is completed in response to the comparison data meeting a preset condition.

Step S360: the geometric parameter value of the preset system is adjusted according to the comparison data in response to the comparison data not meeting the preset condition, and the method is returned to step S330.

As another specific embodiment of the present disclosure, a length direction of the calibration phantom may be perpendicular to a scanning direction of the X-ray source 1, and the calibration phantom is scanned at this angle.

In step S320, the performing X-ray scanning on the calibration phantom includes: scanning the calibration phantom once in response to a size of the calibration phantom being close to a size of the scanning channel of the scanning system; or changing a position of the calibration phantom in the scanning channel and scanning the calibration phantom for a plurality of times in response to the size of the calibration phantom being smaller than a size of the scanning channel of the scanning system. In this way, the calibration phantom may cover the entire scanning region or the designated calibration region range of the CT imaging system as much as possible, so that the effect of image reconstruction within the region range after calibration may reach the overall optimization, which may avoid the significant image quality degradation of the reconstructed image in some certain regions.

Parameters described in step S330 include: a relative position and posture between a light source and a detector in the scanning system. In this way, the fabricated calibration phantom is placed in the scanning channel of the CT imaging system to achieve the scanning and imaging under multiple imaging viewing angles.

In such CT imaging system, the X-ray source 1 and the detector array 2 are relatively stationary and rotate around the detected target 5 for scanning. In order to obtain a reconstructed image of the calibration phantom, the X-ray source and detector array may be set to rotate at different angles for imaging the calibration phantom (for example, scan for 720 times at an interval of 0.5°). In order to obtain a better reconstruction effect, the scanning angle interval may be reduced, or the number of scanning angles may be increased.

The calibration method of the embodiments of the present disclosure is described above with the CT imaging system having the slip ring. Those skilled in the art will understand that the calibration phantom and calibration method of the embodiments of the present disclosure may also be applied to a static CT imaging system. In the static CT imaging system, the X-ray source and the detector array are fixed with respect to the supporting frame without rotation, and the scanning imaging under different viewing angles may be equivalently obtained through a plurality of scanning levels. Considering a difference of reference coordinate systems of different scanning levels, a common practice is to calibrate the geometric parameters of each scanning level separately, and then the coordinate systems of different scanning levels are unified to obtain final calibrated geometric parameters of the system. In order to reduce a amount of data processing, a belt speed of the conveyor may be reduced during a calibration process, so that the calibration wires in each calibration assembly may appear in the same reconstruction slice at the same time.

By using the calibration assembly, the calibration phantom and the calibration method of the present disclosure, by dispersedly connecting the calibration wires of the calibration assembly to the base, and taking advantage of the characteristics that the absorption capacity of the calibration wire for X-rays is greater than the absorption capacity of the base for X-rays, the above-mentioned calibration wires are applied to the calibration phantom, and the calibration phantom is scanned in the scanning system. By continuously adjusting the geometric parameter values in the imaging method, the optimal geometric parameter values that are closest to the real scanning system structure may be obtained, thereby improving the imaging effect of the scanning system. Furthermore, by scanning the calibration phantom for imaging for a plurality of times, and determining whether the scanned comparison data meets the preset condition, the optimal system geometric parameters may be quickly, effectively and accurately obtained, so that the three-dimensional reconstructed image with better image effect may be obtained, other system modules are cooperated to more accurately carry out object recognition and other works, and a better on-site application effect is obtained and customer satisfaction is improved.

Those skilled in the art will understand that features recited in the various embodiments and/or the claims of the present disclosure may be combined and/or incorporated in a variety of ways, even if such combinations or incorporations are not clearly recited in the present disclosure.

The embodiments of the present disclosure have been described above. However, these embodiments are for illustrative purposes only, and are not intended to limit the scope of the present disclosure. Although the various embodiments have been described above separately, this does not mean that the measures in the various embodiments may not be advantageously used in combination. The scope of the present disclosure is defined by the appended claims.

## Claims

1. A calibration phantom, comprising:
a supporting frame (200); and
at least one calibration assembly (100), wherein each of the at least one calibration assembly (100) comprises:
a base (101); and
a plurality of calibration wires (102) dispersedly connected to the base (101), wherein an absorption capacity of the calibration wire (102) for X-rays is greater than an absorption capacity of the base (101) for X-rays, wherein an absorption capacity of the calibration wire (102) for X-rays is greater than an absorption capacity of the supporting frame (200) for X-rays,
**characterized in that**,
the at least one calibration assembly (100) is connected to an inside of the supporting frame (200).

2. The calibration phantom according to claim 1, wherein each of the at least one calibration assembly (100) further comprises two mounting portions (103) arranged at two opposite ends of the base (101).

3. The calibration phantom according to claim 1, wherein a diameter of a smallest circle enclosing a section of the calibration wire (102) falls between 0.5mm and 5 mm.

4. The calibration phantom according to claim 1, wherein the base (101) has a flat plate shape, and the calibration wires (102) are arranged on the base (101) in a length direction of the base (101).

5. The calibration phantom according to any one of the claims 1 to 4, wherein at least one end of the calibration wire (102) protrudes from a side of the base (101); and
wherein the base (101) comprises two sub-bases arranged in parallel, and the calibration wire (102) is connected between the two sub-bases.

6. The calibration phantom according to any one of the claims 1 to 5, wherein each calibration assembly (100) is connected to the inside of the supporting frame (200) through mounting portions (103) arranged at two ends of the calibration assembly (100).

7. The calibration phantom according to any one of the claims 1 to 6, wherein the supporting frame (200) is set as a substantially hexahedral box; and
wherein at least one calibration assembly (100) is arranged in a length direction of the supporting frame (200).

8. The calibration phantom according to claim 7, wherein projections of calibration wires (102) of each calibration assembly (100) in a height direction and/or in a length direction of the supporting frame (200) do not coincide with each other.

9. The calibration phantom according to claim 8, wherein projections of at least two calibration assemblies (100) arranged in the length direction of the supporting (200) frame in the length direction do not coincide with each other; and
wherein projections of the at least two calibration assemblies (100) arranged in the length direction of the supporting frame (200) in the height direction do not coincide with each other.

10. The calibration phantom according to claim 8, wherein projections of at least two calibration assemblies (100) in a transverse direction of the supporting frame (200) intersect with each other, and projections of the at least two calibration assemblies (100) in the length direction of the supporting frame (200) do not coincide with each other.

11. A calibration method, comprising steps of:
placing (S110, S210, S310) the calibration phantom according to any one of the claims 1 to 10 in a scanning channel (6) of a scanning system;
performing (S120, S220, S320) X-ray scanning on the calibration phantom to obtain an imaging result of the calibration phantom;
evaluating (S130, S230) the imaging result according to a preset evaluation method to obtain a first evaluation result;
completing (S140, S240) a calibration of the scanning system in response to the first evaluation result meeting a preset condition; or
adjusting (S150, S250), in response to the first evaluation result not meeting the preset condition, a geometric parameter value of a preset system according to the first evaluation result, and repeating the step of evaluating (S130, S230) the imaging result according to the preset evaluation method.

12. The method according to claim 11, wherein repeating the step of evaluating (S130, S230) the imaging result comprises:
evaluating (S260) the imaging result according to the preset evaluation method to obtain a second evaluation result,
wherein the method further comprises:
completing (S270) the calibration of the scanning system in response to the second evaluation result meeting the preset condition; or
adjusting (S280), in response to the second evaluation result not meeting the preset condition, the geometric parameter value of the preset system according to a reference evaluation result, and repeating the step of evaluating (S260) the imaging result according to the preset evaluation method, wherein the reference evaluation result is characterized as a comparison value between the first evaluation result and the second evaluation result adjacent to the first evaluation result.

13. The method according to claim 11, after the step of performing (S120, S220, S320) X-ray scanning on the calibration phantom to obtain an imaging result of the calibration phantom, further comprising steps of:
simulating (S330) a numerical model of the calibration phantom according to the geometric parameter value of the preset system to obtain a simulation result;
comparing (S340) the simulation result with the imaging result to obtain a comparison data;
completing (S350) the calibration of the scanning system in response to the comparison data meeting the preset condition; or
adjusting (S360), in response to the comparison data not meeting the preset condition, the geometric parameter value of the preset system according to the comparison data, and repeating the step of simulating (S330) a numerical model of the calibration phantom according to the geometric parameter value of the preset system.

14. The method according to any one of claims 11 to 13, wherein a length direction of the calibration phantom is perpendicular to a scanning direction of the calibration phantom;
wherein the step of performing (S320) X-ray scanning on the calibration phantom comprises: scanning the calibration phantom once in response to a size of the calibration phantom being close to a size of the scanning channel of the scanning system; or, changing a position of the calibration phantom in the scanning channel and scanning the calibration phantom for a plurality of times in response to the size of the calibration phantom being smaller than the size of the scanning channel of the scanning system;
wherein the parameter comprises a relative position and a posture between a light source and a detector in the scanning system;
wherein the step of simulating (S330) a numerical model of the calibration phantom according to the geometric parameter value of the preset system is implemented by using one or more of a pure parameter fitting method, a digital radiography, DR, modeling projection simulation and a computed tomography, CT, reconstructed projection simulation;
wherein the simulation result comprises one or more of a projection domain simulation result, a projection domain parametric simulation result, an image domain simulation result and an image domain parametric simulation result; and
wherein the imaging result comprises one or more of a scanning data, a DR image, a slice image and a three-dimensional reconstructed image.

## Patentansprüche

1. Kalibrierungsphantom, umfassend:
einen Halterahmen (200); und
mindestens eine Kalibrierungsanordnung (100), wobei jede der mindestens einen Kalibrierungsanordnung (100) Folgendes umfasst:
eine Basis (101); und
eine Vielzahl von Kalibrierungsdrähten (102), die verstreut mit der Basis (101) verbunden sind, wobei ein Absorptionsvermögen des Kalibrierungsdrahts (102) für Röntgenstrahlen größer ist als ein Absorptionsvermögen der Basis (101) für Röntgenstrahlen, wobei ein Absorptionsvermögen des Kalibrierungsdrahts (102) für Röntgenstrahlen größer ist als ein Absorptionsvermögen des Halterahmens (200) für Röntgenstrahlen,
**dadurch gekennzeichnet, dass**
die mindestens eine Kalibrierungsanordnung (100) mit einer Innenseite des Halterahmens (200) verbunden ist.

2. Kalibrierungsphantom nach Anspruch 1, wobei jede der mindestens einen Kalibrierungsanordnung (100) ferner zwei Montageabschnitte (103) umfasst, die an zwei gegenüberliegenden Enden der Basis (101) angeordnet sind.

3. Kalibrierungsphantom nach Anspruch 1, wobei der Durchmesser des kleinsten Kreises, der einen Bereich des Kalibrierungsdrahts (102) umschließt, zwischen 0,5 mm und 5 mm ist.

4. Kalibrierungsphantom nach Anspruch 1, wobei die Basis (101) eine flache Plattenform aufweist und die Kalibrierungsdrähte (102) in einer Längsrichtung der Basis (101) auf der Basis (101) angeordnet sind.

5. Kalibrierungsphantom nach einem der Ansprüche 1 bis 4, wobei mindestens ein Ende des Kalibrierungsdrahts (102) von einer Seite der Basis (101) hervorsteht; und
wobei die Basis (101) zwei parallel angeordnete Teilbasen umfasst und der Kalibrierungsdraht (102) zwischen den beiden Teilbasen verbunden ist.

6. Kalibrierungsphantom nach einem der Ansprüche 1 bis 5, wobei jede Kalibrierungsanordnung (100) mit der Innenseite des Halterahmens (200) durch Montageabschnitte (103) verbunden ist, die an zwei Enden der Kalibrierungsanordnung (100) angeordnet sind.

7. Kalibrierungsphantom nach einem der Ansprüche 1 bis 6, wobei der Halterahmen (200) als ein im Wesentlichen hexaedrischer Kasten eingerichtet ist; und
wobei mindestens eine Kalibrierungsanordnung (100) in einer Längsrichtung des Halterahmens (200) angeordnet ist.

8. Kalibrierungsphantom nach Anspruch 7, wobei Vorsprünge der Kalibrierungsdrähte (102) von jeder Kalibrierungsanordnung (100) in einer Höhenrichtung und/oder in einer Längsrichtung des Halterahmens (200) nicht miteinander übereinstimmen.

9. Kalibrierungsphantom nach Anspruch 8, wobei die Vorsprünge von mindestens zwei Kalibrierungsanordnungen (100), die in der Längsrichtung des Halterahmens (200) angeordnet sind, nicht miteinander übereinstimmen; und
wobei die Vorsprünge der mindestens zwei in Längsrichtung des Halterahmens (200) angeordneten Kalibrierungsanordnungen (100) in Höhenrichtung nicht miteinander übereinstimmen.

10. Kalibrierungsphantom nach Anspruch 8, wobei sich Projektionen von mindestens zwei Kalibrierungsanordnungen (100) in einer Querrichtung des Halterahmens (200) überschneiden und Projektionen der mindestens zwei Kalibrierungsanordnungen (100) in der Längsrichtung des Halterahmens (200) nicht miteinander übereinstimmen.

11. Kalibrierungsverfahren, umfassend folgende Schritte:
Platzieren (S110, S210, S310) des Kalibrierungsphantoms nach einem der Ansprüche 1 bis 10 in einem Scan-Kanal (6) eines Scan-Systems;
Durchführen (S120, S220, S320) von Röntgen-Scan an dem Kalibrierungsphantom, um ein Bildgebungsresultat des Kalibrierungsphantoms zu erlangen;
Bewerten (S130, S230) des Bildgebungsresultats gemäß einem voreingestellten Bewertungsverfahren, um ein erstes Bewertungsresultat zu erlangen;
Abschließen (S140, S240) einer Kalibrierung des Scan-Systems als Reaktion darauf, dass das erste Bewertungsresultat eine voreingestellte Bedingung erfüllt; oder
Anpassen (S150, S250), als Reaktion darauf, dass das erste Bewertungsresultat die voreingestellte Bedingung nicht erfüllt, eines geometrischen Parameterwerts eines voreingestellten Systems gemäß dem ersten Bewertungsresultat und Wiederholen des Schritts eines Bewertens (S130, S230) des Bildgebungsresultats gemäß dem voreingestellten Bewertungsverfahren.

12. Verfahren nach Anspruch 11, wobei das Wiederholen des Schritts eines Bewertens (S130, S230) des Bildgebungsresultats Folgendes umfasst:
Bewerten (S260) des Bildgebungsresultats gemäß dem voreingestellten Bewertungsverfahren, um ein zweites Bewertungsresultat zu erlangen,
wobei das Verfahren ferner Folgendes umfasst:
Abschließen (S270) der Kalibrierung des Scan-Systems als Reaktion darauf, dass das zweite Bewertungsresultat die voreingestellte Bedingung erfüllt; oder
Anpassen (S280), als Reaktion darauf, dass das zweite Bewertungsresultat die voreingestellte Bedingung nicht erfüllt, des geometrischen Parameterwerts des voreingestellten Systems gemäß einem Referenzbewertungsresultat und Wiederholen des Schritts eines Bewertens (S260) des Bildgebungsresultats gemäß dem voreingestellten Bewertungsverfahren, wobei das Referenzbewertungsresultat als ein Vergleichswert zwischen dem ersten Bewertungsresultat und dem ersten Bewertungsresultat angrenzend an das zweite Bewertungsresultat gekennzeichnet ist.

13. Verfahren nach Anspruch 11, nach dem Schritt eines Durchführen (S120, S220, S320) von Röntgen-Scan an dem Kalibrierungsphantom, um ein Bildgebungsresultat des Kalibrierungsphantoms zu erlangen, ferner umfassend die folgenden Schritte:
Simulieren (S330) eines numerischen Modells des Kalibrierungsphantoms gemäß dem geometrischen Parameterwert des voreingestellten Systems, um ein Simulationsresultat zu erlangen;
Vergleichen (S340) des Simulationsresultats mit dem Bildgebungsresultat, um Vergleichsdaten zu erlangen;
Abschließen (S350) der Kalibrierung des Scan-Systems als Reaktion darauf, dass die Vergleichsdaten die voreingestellte Bedingung erfüllen; oder
Anpassen (S360), als Reaktion darauf, dass die Vergleichsdaten die voreingestellte Bedingung nicht erfüllen, des geometrischen Parameterwerts des voreingestellten Systems gemäß den Vergleichsdaten, und Wiederholen des Schritts eines Simulierens (S330) eines numerischen Modells des Kalibrierungsphantoms gemäß dem geometrischen Parameterwert des voreingestellten Systems.

14. Verfahren nach einem der Ansprüche 11 bis 13, wobei eine Längsrichtung des Kalibrierungsphantoms senkrecht zu einer Scan-Richtung des Kalibrierungsphantoms ist;
wobei der Schritt eines Durchführens (S320) von Röntgen-Scan an dem Kalibrierungsphantom Folgendes umfasst:
einmaliges Scannen des Kalibrierungsphantoms als Reaktion darauf, dass eine Größe des Kalibrierungsphantoms nahe einer Größe des Scan-Kanals des Scan-Systems ist; oder
Ändern einer Position des Kalibrierungsphantoms in dem Scan-kanal und mehrmaliges Scannen des Kalibrierungsphantoms als Reaktion darauf, dass die Größe des Kalibrierungsphantoms kleiner ist als die Größe des Scan-Kanals des Abtastsystems;
wobei der Parameter eine relative Position und eine Stellung zwischen einer Lichtquelle und einem Detektor in dem Scan-System umfasst;
wobei der Schritt eines Simulierens (S330) eines numerischen Modells des Kalibrierungsphantoms gemäß dem geometrischen Parameterwert des voreingestellten Systems unter Verwendung eines oder mehrerer von einem reinen Parameteranpassungsverfahren, einer digitalen Radiographie (DR), die eine Projektionssimulation modelliert, und einer Computertomographie (CT), die eine rekonstruierte Projektion simuliert, implementiert wird;
wobei das Simulationsresultat eines oder mehrerer von einem Projektionsbereich-Simulationsresultat, einem parametrischen Projektionsbereich-Simulationsresultat, einem Bildbereich-Simulationsresultat und einem parametrischen Bildbereich-Simulationsresultat umfasst; und
wobei das Bildgebungsresultat eines oder mehrere von Scan-Daten, ein DR-Bild, ein Schichtbild und ein dreidimensional rekonstruiertes Bild umfasst.

## Revendications

1. Fantôme de calibrage, comprenant :
un châssis de support (200) ; et
au moins un ensemble de calibrage (100), dans lequel chacun de l'au moins un ensemble de calibrage (100) comprend :
une base (101) ; et
une pluralité de fils de calibrage (102) reliés de manière dispersée à la base (101), dans lequel une capacité d'absorption du fil de calibrage (102) pour les rayons X est supérieure à une capacité d'absorption de la base (101) pour les rayons X, dans lequel une capacité d'absorption du fil de calibrage (102) pour les rayons X est supérieure à une capacité d'absorption du châssis de support (200) pour les rayons X,
**caractérisé en ce que**
l'au moins un ensemble de calibrage (100) est relié à un intérieur du châssis de support (200).

2. Fantôme de calibrage selon la revendication 1, dans lequel chacun de l'au moins un ensemble de calibrage (100) comprend en outre deux parties de montage (103) prévues à deux extrémités opposées de la base (101).

3. Fantôme de calibrage selon la revendication 1, dans lequel un diamètre d'un cercle le plus petit qui contient une section du fil de calibrage (102) est compris entre 0,5 mm et 5 mm.

4. Fantôme de calibrage selon la revendication 1, dans lequel la base (101) possède une forme de plaque plane, et les fils de calibrage (102) sont prévus sur la base (101) dans le sens de la longueur de la base (101).

5. Fantôme de calibrage selon l'une quelconque des revendications 1 à 4, dans lequel au moins une extrémité du fil de calibrage (102) dépasse depuis un côté de la base (101) ; et
dans lequel la base (101) comprend deux sous-bases prévues en parallèle, et le fil de calibrage (102) est relié entre les deux sous-bases.

6. Fantôme de calibrage selon l'une quelconque des revendications 1 à 5, dans lequel chaque ensemble de calibrage (100) est relié à l'intérieur du châssis de support (200) par le biais des parties de montage (103) prévues aux deux extrémités de l'ensemble de calibrage (100).

7. Fantôme de calibrage selon l'une quelconque des revendications 1 à 6, dans lequel le châssis de support (200) est défini comme un boîtier sensiblement hexaèdre ; et
dans lequel au moins un ensemble de calibrage (100) est prévu dans le sens de la longueur du châssis de support (200).

8. Fantôme de calibrage selon la revendication 7, dans lequel les saillies des fils de calibrage (102) de chaque ensemble de calibrage (100) dans le sens de la hauteur et/ou dans le sens de la longueur du châssis de support (200) ne coïncident pas les unes avec les autres.

9. Fantôme de calibrage selon la revendication 8, dans lequel les saillies d'au moins deux ensembles de calibrage (100) prévues dans le sens de la longueur du châssis de support (200) dans le sens de la longueur ne coïncident pas les unes avec les autres ; et
dans lequel les saillies des au moins deux ensembles de calibrage (100) prévues dans le sens de la longueur du châssis de support (200) dans le sens de la hauteur ne coïncident pas les unes avec les autres.

10. Fantôme de calibrage selon la revendication 8, dans lequel les saillies d'au moins deux ensembles de calibrage (100) dans une direction transversale du châssis de support (200) se croisent, et les saillies des au moins deux ensembles de calibrage (100) dans le sens de la longueur du châssis de support (200) ne coïncident pas les unes avec les autres.

11. Procédé de calibrage, comprenant les étapes consistant à :
placer (S110, S210, S310) le fantôme de calibrage selon l'une quelconque des revendications 1 à 10 dans un canal de balayage (6) d'un système de balayage ;
effectuer (S120, S220, S320) un balayage par rayons X sur le fantôme de calibrage afin d'obtenir un résultat d'imagerie du fantôme de calibrage ;
évaluer (S130, S230) le résultat d'imagerie selon un procédé d'évaluation prédéfini afin d'obtenir un premier résultat d'évaluation ;
réaliser (S140, S240) un calibrage du système de balayage en réponse au premier résultat d'évaluation qui satisfait une condition prédéfinie ; ou
ajuster (S150, S250), en réponse au premier résultat d'évaluation qui ne satisfait pas la condition prédéfinie, une valeur de paramètre géométrique d'un système prédéfini selon le premier résultat d'évaluation, et répéter l'étape d'évaluation (S130, S230) du résultat d'imagerie selon le procédé d'évaluation prédéfini.

12. Procédé selon la revendication 11, dans lequel la répétition de l'étape d'évaluation (S130, S230) du résultat d'imagerie comprend :
l'évaluation (S260) du résultat d'imagerie selon le procédé d'évaluation prédéfini afin d'obtenir un deuxième résultat d'évaluation,
dans lequel le procédé comprend en outre :
la réalisation (S270) du calibrage du système de balayage en réponse au deuxième résultat d'évaluation qui satisfait la condition prédéfinie ; ou
l'ajustement (S280), en réponse au deuxième résultat d'évaluation qui ne satisfait pas la condition prédéfinie, de la valeur de paramètre géométrique du système prédéfini selon un résultat d'évaluation de référence, et la répétition de l'étape d'évaluation (S260) du résultat d'imagerie selon le procédé d'évaluation prédéfini, dans lequel le résultat d'évaluation de référence est caractérisé comme une valeur de comparaison entre le premier résultat d'évaluation et le deuxième résultat d'évaluation adjacent au premier résultat d'évaluation.

13. Procédé selon la revendication 11, après l'étape d'exécution (S120, S220, S320) du balayage par rayons X sur le fantôme de calibrage afin d'obtenir un résultat d'imagerie du fantôme de calibrage, comprenant en outre les étapes consistant à :
simuler (S330) un modèle numérique du fantôme de calibrage selon la valeur de paramètre géométrique du système prédéfini afin d'obtenir un résultat de simulation ;
comparer (S340) le résultat de simulation avec le résultat d'imagerie afin d'obtenir des données de comparaison ;
réaliser (S350) le calibrage du système de balayage en réponse aux données de comparaison qui satisfont la condition prédéfinie ; ou
ajuster (S360), en réponse aux données de comparaison qui ne satisfont pas la condition prédéfinie, la valeur de paramètre géométrique du système prédéfini selon les données de comparaison, et répéter l'étape de simulation (S330) d'un modèle numérique du fantôme de calibrage selon la valeur de paramètre géométrique du système prédéfini.

14. Procédé selon l'une quelconque des revendications 11 à 13, dans lequel un sens de la longueur du fantôme de calibrage est perpendiculaire à une direction de balayage du fantôme de calibrage ;
dans lequel l'étape d'exécution (S320) d'un balayage par rayons X sur le fantôme de calibrage comprend : le balayage du fantôme de calibrage une fois en réponse à une taille du fantôme de calibrage qui est proche d'une taille du canal de balayage du système de balayage ; ou le changement d'une position du fantôme de calibrage dans le canal de balayage et le balayage du fantôme de calibrage une pluralité de fois en réponse à la taille du fantôme de calibrage qui est inférieure à la taille du canal de balayage du système de balayage ;
dans lequel le paramètre comprend une position relative et une posture entre une source de lumière et un détecteur dans le système de balayage ;
dans lequel l'étape de simulation (S330) d'un modèle numérique du fantôme de calibrage selon la valeur de paramètre géométrique du système prédéfini est effectuée à l'aide d'un ou plusieurs d'un procédé d'ajustement de paramètre pur, d'une simulation de projection par modélisation par radiographie numérique, DR, et d'une simulation de projection reconstruite par tomographie assistée par ordinateur, CT ;
dans lequel le résultat de simulation comprend un ou plusieurs d'un résultat de simulation dans un domaine de projection, d'un résultat de simulation paramétrique dans un domaine de projection, d'un résultat de simulation dans un domaine d'image et d'un résultat de simulation paramétrique dans un domaine d'image ; et
dans lequel le résultat d'imagerie comprend un ou plusieurs de données de balayage, d'une image de radiographie numérique, d'une image en coupe et d'une image reconstruite en trois dimensions.
